# EUROPEAN PATENT APPLICATION

(11) **EP 4 641 187 A1**
(43) Date of publication of application: **29.10.2025**
(21) Application number: 25172180.9
(22) Date of filing: 24.04.2025
(51) Int. Cl.: G01N 25/18, G01N 27/18, G01N 33/00

(54) **APPARATUS AND PROCESS FOR PARAHYDROGEN CONCENTRATION ANALYSIS**

(30) Priority: 25.04.2024 US 202418645978
(71) Applicant: Air Products and Chemicals, Inc., Allentown, PA 18106 (US)
(72) Inventor: MACCONNELL, Matthew H, Orefield, 18069 (US)
(74) Representative: Beck Greener LLP

(57) **Abstract**

An apparatus and process for parahydrogen concentration analysis can include forming a synthetic gas that can mimic a hydrogen fluid (e.g., gas or liquid) having 0 mole percent (mol%) parahydrogen. An analyzer (6) may then be calibrated *via* use of a reference hydrogen gas having a first pre-selected parahydrogen concentration (e.g., a 25 mol% parahydrogen content) and the synthetic gas to identify multiple calibration points for the analyzer (6) for calibration of the analyzer (6). After the analyzer (6) is calibrated, it can subsequently be utilized to determine a parahydrogen concentration of one or more samples of hydrogen fluid. Some embodiments can be utilized to help ensure that a liquid hydrogen that is produced *via* liquefaction or other liquid hydrogen production process has a content of at least 95 mol% parahydrogen.

## Description

The present innovation relates to processes and apparatuses for analysis of hydrogen fluid (*e.g*., liquid hydrogen, hydrogen gas, *etc*.) for determination of a concentration of parahydrogen within the hydrogen (also referred to herein as H₂ or H2).

Conventional approaches for evaluation of the parahydrogen and orthohydrogen concentrations within hydrogen fluid typically involves use of an analyzer that is calibrated by passing that gas through a catalyst tube to adjust the parahydrogen content of the fluid and then assigning a parahydrogen concentration to a gas for analysis by the analyzer.

Examples of some approaches for parahydrogen and/or orthohydrogen analysis can be appreciated from Japanese Patent No. JP 6327656. Other approaches can include use of a nuclear magnetic moment difference as disclosed in Chinese Patent No. CN104730141.

The inventor has determined that many conventional approaches for calibration of analysis equipment for evaluation of a parahydrogen content of hydrogen fluid (*e.g*., liquid hydrogen) have a number of shortcomings. For instance, in many approaches that utilize a catalyst tube approach for adjustment of the concentration of parahydrogen content in a gas to be used for calibration purposes, it is often not possible to determine if the hydrogen is at 100% of equilibrium for a given temperature or some percentage approaching full equilibrium. The effectiveness of the catalyst approach often depends on periodic reactivation and plant designs for manufacture of the hydrogen may utilize a different target conversion (*e.g.,* a target conversion below 100 mole percent (mol%) parahydrogen).

Also, the inventor has determined that the actual parahydrogen value is a strong function of temperature and, in the catalyst tube based approaches, the temperature for a calibration gas that is being utilized may not be known. For instance, the subcooling of liquid hydrogen can be a variable and there may not be a known temperature measurement sufficiently accurate to use for the lookup of a parahydrogen content value based on a particular temperature.

These types of issues can be particularly problematic for analyzers that rely upon a thermal conductivity based detection scheme for detecting a concentration of parahydrogen and/or orthohydrogen within a hydrogen gas sample. Often, these devices can rely upon a pre-defined calibration curve or calibration scheme. In the event one of the calibration gas reference points is wrong, this can significantly affect the accuracy of the analyzer's output for a particular sample. This type of problem can be particularly serious in situations where a precise and accurate determination of the parahydrogen content in a hydrogen fluid is desired (*e.g.,* for certification of liquid hydrogen that may be supplied for use in aerospace applications, providing liquid hydrogen for use as a fuel in rocket launching or other aerospace related uses, *etc.*)

The inventor has surprisingly found that current approaches for conventionally analyzing parahydrogen content often do not address the questions of the extent of reaching para equilibrium or the liquid H2 temperature of this calibration fluid source. This approach fails to use any type of reference to a recognized standard and instead simply asserts an assumed concentration of parahydrogen. This is not an accurate second calibration point for use in calibration of an analyzer. The inventor does not believe this type of problem is even appreciated conventionally as it is typically assumed that the conventional approach is suitable and accurate.

Also, the utilization of catalyst based approaches can incur substantial capital costs and operational costs that can constrain operations. For example, the catalyst material can add tens of thousands of dollars in cost to the parahydrogen analysis scheme that may be utilized due to the cost of the liquid hydrogen assembly containing the catalyst tube. Also, the use of the catalyst tube incurs substantial work and cost in terms of maintenance (*e.g.,* replacing expensive catalyst material, regeneration of the catalyst material, *etc*.)*.* These types of issues add operational complexity and capital cost to liquid hydrogen analytical systems while implementing an ambiguous calibration standard needed for certifying hydrogen product parahydrogen content of the produced hydrogen as a product to a customer.

The inventor has developed new processes and apparatus schemes for providing a different approach for parahydrogen content analysis. Embodiments can be provided to improve operational flexibility, reduce the amount of time needed to perform calibrations of analyzer equipment, can provide reduced maintenance requirements, can avoid use of more expensive catalyst tube based equipment, and can provide a more reliable approach for calibration with use of calibration reference fluids that can enable more accurate measurement of parahydrogen concentrations.

In some embodiments, a multi-point calibration can be utilized that includes use of a first calibration fluid. This first calibration fluid can be created to function as a synthetic 0 mol% p-H2 hydrogen fluid composition (*e.g.,* a fluid that is to mimic a 0 mol% p-H2 hydrogen gas). The fluid that can mimic a 0 mol% p-H2 hydrogen gas can be, for example, a gas mixture that includes hydrogen gas mixed with at least one other gas so that the thermal conductivity of the gas is the same or substantially similar to a 0 mol% p-H2 hydrogen gas. As another example, the fluid that can mimic a 0 mol% p-H2 hydrogen gas can be, for example, a gas mixture that includes liquid hydrogen mixed with at least one other gas (*e.g.,* helium, krypton, *etc*.) so that the thermal conductivity of the gas is the same or substantially similar to a 0 mol% p-H2 hydrogen gas.

The multi-point calibration can also utilize a second calibration fluid that may be considered a "span fluid." The second calibration fluid can be a room temperature hydrogen fluid that is known to be at an established 25% parahydrogen (p-H2) concentration, for example. This second calibration fluid can be a 25 mol% p-H2 hydrogen stored in a storage vessel (*e.g.,* cylinder) for example. In some embodiments, the second calibration fluid can be considered a "span gas".

Some embodiments can utilize an analyzer that is configured as a thermal conductivity analyzer. For example, a calibration of the thermal conductivity analyzer using first and second calibration fluids (*e.g.,* a synthetic 0 mol% p-H2 gas and a 25 mol% p-H2 gas, another type of p-H2 content hydrogen fluid and a synthetic 0 mol% p-H2 fluid, *etc*.) can be provided to set the calibration slope of the analyzer to enable measurement of a p-H2 value for a sample obtained from produced liquid hydrogen such that the calibration slope is able to be more accurately extrapolate up to a 100% p-H2 concentration. The inventor has also conducted experimentation that has helped confirm that the accuracy of this different type of calibration technique can be relied upon and be provided in a precise manner. The experimental results show that embodiments can provide accurate parahydrogen concentration determinations that involve less costly and arbitrary calibration processing.

Also, the inventor has found that some embodiments of my apparatus and process can utilize conventional analyzer equipment (*e.g.,* thermal conductivity analyzers that can be provided by Teledyne Analytical Instruments, *etc*.)*,* which can permit embodiments to be utilized more easily, efficiently, and at a lower operational cost and capital cost as compared to conventional approaches. Embodiments can provide quicker, more efficient, and reliable calibration that can facilitate improved and more reliable analysis for parahydrogen and/or orthohydrogen concentrations of hydrogen fluid while also providing such features at lower operational and capital costs.

In a first aspect, a process for parahydrogen concentration analysis can be provided. The process can include creating a synthetic gas to mimic a 0 mole percent (mol%) parahydrogen content hydrogen fluid such that the synthetic gas has a thermal conductivity that is equivalent to a thermal conductivity of a hydrogen fluid having a 0 mol% parahydrogen content, and calibrating an analyzer using the synthetic gas as a first calibration fluid and a second calibration fluid during the calibrating of the analyzer. After the calibrating of the analyzer, a reference gas can be fed to the analyzer and a fluid of a sample of hydrogen fluid can be fed to the analyzer for a determination of a parahydrogen content of the sample and/or an orthohydrogen content of the sample.

In some embodiments, the process can be a process for calibration of an analyzer that is used for parahydrogen concentration analysis. In such embodiments, the process can include creating a synthetic gas to mimic a 0 mole percent (mol%) parahydrogen content hydrogen fluid such that the synthetic gas has a thermal conductivity that is equivalent to a thermal conductivity of a hydrogen fluid having a 0 mol% parahydrogen content, and calibrating an analyzer using the synthetic gas as a first calibration fluid and a second calibration fluid during the calibrating of the analyzer. The calibrated analyzer can then be utilized in evaluation of one or more samples of hydrogen fluid for determination of the parahydrogen content of the sample(s) and/or the orthohydrogen content of the sample(s).

In some embodiments, the analyzer can be a thermal conductivity analyzer. The analyzer may alternatively be another type of suitable analyzer.

The thermal conductivity of the synthetic gas can be equivalent to the thermal conductivity of a hydrogen fluid having a 0 mol% parahydrogen content by the synthetic gas having a thermal conductivity that is equal to the thermal conductivity of the hydrogen fluid having the 0 mol% parahydrogen content. The thermal conductivity of the synthetic gas can also be equivalent to the thermal conductivity of a hydrogen fluid having a 0 mol% parahydrogen content by the synthetic gas having a thermal conductivity that is within 1% of the thermal conductivity of the hydrogen fluid having the 0 mol% parahydrogen content. The thermal conductivity of the synthetic gas can also be equivalent to the thermal conductivity of a hydrogen fluid having a 0 mol% parahydrogen content by the synthetic gas having a thermal conductivity that is within 0.5% of the thermal conductivity of the hydrogen fluid having the 0 mol% parahydrogen content. The thermal conductivity of the synthetic gas can also be equivalent to the thermal conductivity of a hydrogen fluid having a 0 mol% parahydrogen content by the synthetic gas having a thermal conductivity that is within 0.1% of the thermal conductivity of the hydrogen fluid having the 0 mol% parahydrogen content. As yet another example, the thermal conductivity of the synthetic gas can also be equivalent to the thermal conductivity of a hydrogen fluid having a 0 mol% parahydrogen content by the synthetic gas having a thermal conductivity that is within 0.01% of the thermal conductivity of the hydrogen fluid having the 0 mol% parahydrogen content.

In a second aspect, the creating of the synthetic gas can include mixing a 25 mol% parahydrogen content hydrogen gas with helium gas to form the synthetic gas such that the synthetic gas has a helium content of between 0.5 mol% helium and 4 mol% helium and a balance of the synthetic gas being the 25 mol% parahydrogen content hydrogen gas. For example, in some embodiments the helium content of the synthetic gas can be between 1.15 mol% helium and 1.30 mol% helium. In other embodiments, the helium content of the synthetic gas can be between 1.17 mol% helium and 1.27 mol% helium.

For the avoidance of doubt, when a range is expressed in this specification in terms of being "between" a lower limit "and" an upper limit, the limits are included in the range. By way of example, a synthetic gas having a helium content of between 0.5 mol% helium and 4 mol% helium is equivalent to a synthetic gas having a helium content in a range from 0.5 mol% helium to 4 mol% helium.

In a third aspect, the creating of the synthetic gas can include mixing a 25 mol% parahydrogen content hydrogen gas with at least one additive fluid to form the synthetic gas. The at least one additive fluid can include one or more of: nitrogen gas, krypton gas, argon gas, helium gas, or a combination thereof in some embodiments.

In a fourth aspect, the creating of the synthetic gas can include mixing a hydrogen gas with at least one additive fluid to form the synthetic gas. The at least one additive fluid can be mixed with the hydrogen gas so that the thermal conductivity of the synthetic gas that is formed can be equivalent to the thermal conductivity of a hydrogen fluid having a 0 mol% parahydrogen content.

In a fifth aspect, a process for performing a calibration for parahydrogen concentration analysis can be provided. The process can include creating a synthetic gas to mimic a 0 mol% parahydrogen content hydrogen fluid such that the synthetic gas has a thermal conductivity that is equivalent to a thermal conductivity of a hydrogen fluid having a 0 mol% parahydrogen content and calibrating an analyzer using the synthetic gas as a first calibration fluid and a second calibration fluid.

In some embodiments, the calibrating of the analyzer using the synthetic gas as the first calibration fluid and the second calibration fluid can include feeding of the first calibration fluid and a reference fluid to the analyzer for being fed into and/or through measurement cells of the analyzer for a pre-selected equilibrium time period for establishing a first calibration data point for the analyzer. After the first calibration point is set on the analyzer *via* use of the first calibration fluid, the feeding of the first calibration fluid to the analyzer can be stopped and the second calibration fluid and the reference fluid can subsequently be fed to the analyzer for a pre-selected equilibrium time period for establishing a second calibration data point for the analyzer.

The calibrating of the analyzer using the synthetic gas as the first calibration fluid and the second calibration fluid can also include entering input into the analyzer to define a parahydrogen content of 0 mol% parahydrogen for the first calibration fluid while the first calibration fluid is fed to the analyzer to establish the first calibration data point and entering input into the analyzer to define a parahydrogen content of the second calibration fluid while the second calibration fluid is fed to the analyzer to establish the second calibration data point.

In some embodiments, the parahydrogen content of the second calibration fluid is 25 mol% parahydrogen and the second calibration fluid is a hydrogen gas. In other embodiments, the parahydrogen content of the second calibration fluid can be a hydrogen gas having another pre-selected parahydrogen concentration.

As discussed above, the creating of the synthetic gas can include mixing a 25 mol% parahydrogen content hydrogen gas with at least one additive fluid to form the synthetic gas. The at least one additive fluid can include one or more of: nitrogen gas, krypton gas, argon gas, helium gas, or a combination thereof. In some embodiments, the helium content of the synthetic gas can be between 1.15 mol% helium and 1.30 mol% helium or be between 1.17 mol% helium and 1.27 mol% helium.

In a sixth aspect, the process of the first aspect or the fifth aspect can include other features or elements. Examples of such features or elements can be appreciated from the exemplary embodiments of the processes discussed herein and/or shown in the drawings. Embodiments of the process can also utilize elements of an apparatus for parahydrogen concentration analysis.

In a seventh aspect, an apparatus for parahydrogen concentration analysis is provided. An embodiment of the apparatus can be configured to implement an embodiment of a process for calibrating an analyzer and/or an embodiment of a process for parahydrogen concentration analysis. Embodiments of the apparatus can include an analyzer, a source of a reference gas that is fluidly connectable to the analyzer, a source of a first calibration gas that is fluidly connectable to the analyzer, and a source of a second calibration fluid comprising hydrogen that is fluidly connectable to the analyzer. The first calibration gas can be a synthetic gas that has a content of hydrogen and at least one additive fluid to mimic a 0 mole percent (mol%) parahydrogen content hydrogen fluid such that the synthetic gas has a thermal conductivity that is equivalent to a thermal conductivity of a hydrogen fluid having a 0 mol% parahydrogen content.

As noted above, the analyzer can be a thermal conductivity analyzer in some embodiments. The analyzer may alternatively be another type of suitable analyzer.

As mentioned above, the thermal conductivity of the synthetic gas can be equivalent to the thermal conductivity of a hydrogen fluid having a 0 mol% parahydrogen content by the synthetic gas having a thermal conductivity that is equal to the thermal conductivity of the hydrogen fluid having the 0 mol% parahydrogen content. The thermal conductivity of the synthetic gas can also be equivalent to the thermal conductivity of a hydrogen fluid having a 0 mol% parahydrogen content by the synthetic gas having a thermal conductivity that is within 1% of the thermal conductivity of the hydrogen fluid having the 0 mol% parahydrogen content. The thermal conductivity of the synthetic gas can also be equivalent to the thermal conductivity of a hydrogen fluid having a 0 mol% parahydrogen content by the synthetic gas having a thermal conductivity that is within 0.5% of the thermal conductivity of the hydrogen fluid having the 0 mol% parahydrogen content. The thermal conductivity of the synthetic gas can also be equivalent to the thermal conductivity of a hydrogen fluid having a 0 mol% parahydrogen content by the synthetic gas having a thermal conductivity that is within 0.1% of the thermal conductivity of the hydrogen fluid having the 0 mol% parahydrogen content. As yet another example, the thermal conductivity of the synthetic gas can also be equivalent to the thermal conductivity of a hydrogen fluid having a 0 mol% parahydrogen content by the synthetic gas having a thermal conductivity that is within 0.01% of the thermal conductivity of the hydrogen fluid having the 0 mol% parahydrogen content.

The source of the reference gas, first calibration fluid and second calibration fluid can include a storage container, storage vessel, or array of such storage containers or vessels. For example, each source can include one or more cylinders or other type of fluid storage canister.

In an eighth aspect, embodiments of the apparatus can include other features or other elements. Examples of such features and/or elements can include features or elements of exemplary embodiments discussed herein and/or shown in the drawings.

It should be appreciated that embodiments of the process and apparatus can utilize various conduit arrangements and process control elements. The embodiments may utilize sensors (e.g., pressure sensors, temperature sensors, flow rate sensors, concentration sensors, *etc.),* controllers, valves, piping, and other process control elements. Some embodiments can utilize an automated process control system and/or a distributed control system (DCS), for example. Various different conduit arrangements and process control systems can be utilized to meet a particular set of design criteria.

Other details, objects, and advantages of the apparatus for parahydrogen concentration analysis, process for parahydrogen concentration analysis, process for performing a calibration for parahydrogen concentration analysis, a process for forming a synthetic gas to mimic a 0 mol% parahydrogen content hydrogen gas, and methods of making and using the same will become apparent as the following description of certain exemplary embodiments thereof proceeds.

Exemplary embodiments of our apparatus for parahydrogen concentration analysis, a process for parahydrogen concentration analysis, a process for performing a calibration for parahydrogen concentration analysis, a process for forming a synthetic gas to mimic a 0 mol% parahydrogen content hydrogen gas, and methods of making and using the same are shown in the drawings included herewith. It should be understood that like reference characters used in the drawings may identify like components.

Figure 1 is a block diagram of a first exemplary embodiment of an apparatus for parahydrogen concentration analysis. An exemplary embodiment of a process for parahydrogen concentration analysis can also be appreciated from Figure 1.

Figure 2 is a block diagram of an exemplary embodiment of tan analyzer 6 that can be utilized in the exemplary embodiment of the apparatus for parahydrogen concentration analysis shown in Figure 1.

Figure 3 is a graph illustrating an equilibrium curve for parahydrogen concentrations at different temperatures ranging from 0 Kelvin to 300 Kelvin (K).

Figure 4 is a flow chart illustrating an exemplary embodiment of a process for parahydrogen concentration analysis. This process can also utilize an exemplary embodiment of a process for performing a calibration for parahydrogen concentration analysis. The first exemplary embodiments of the apparatus for parahydrogen concentration analysis can implement these exemplary embodiments of these processes.

Figure 5 is a schematic illustration of an exemplary embodiment of a process for forming a synthetic gas to mimic a 0 mol% parahydrogen content hydrogen gas.

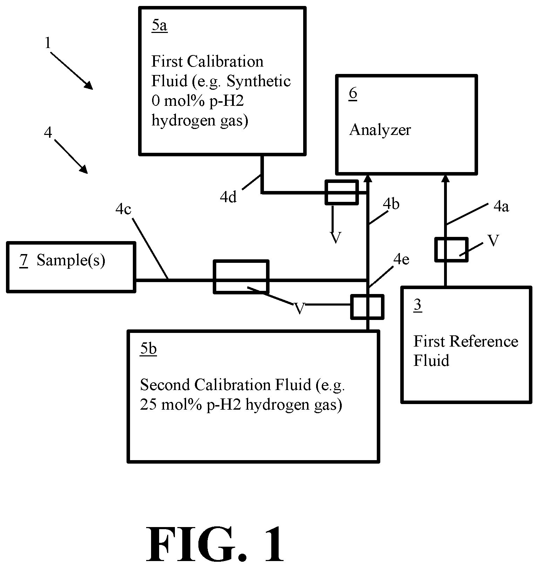

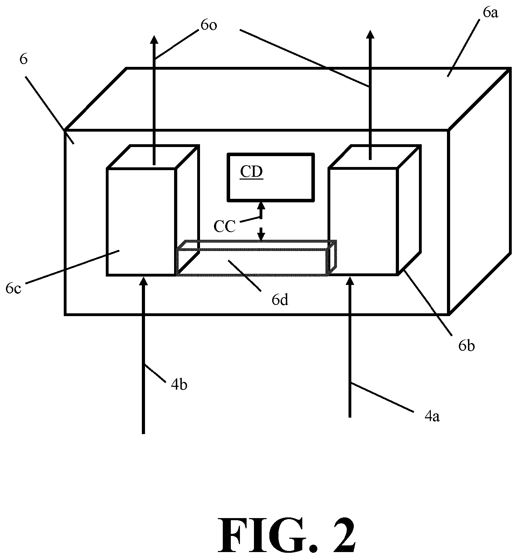

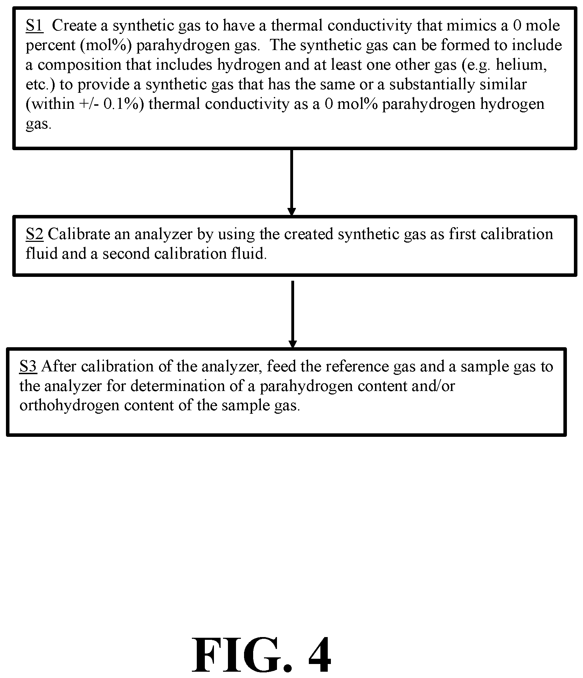

Referring to Figures 1-5, an apparatus 1 for parahydrogen concentration analysis can include an analyzer 6 that is positioned to receive a first reference fluid from a source of first reference gas 3 and either (i) a sample gas from a source of a sample fluid 7, (ii) a source of a first calibration fluid 5a, or (iii) a source of a second calibration fluid 5b. The source of the first calibration fluid 5a can be at least one storage vessel (*e.g.,* a cylinder or other pressurized storage vessel) that retains a synthetic 0 mol% parahydrogen gas, for example. The source of the second calibration fluid 5b can be at least one storage vessel (*e.g.,* a cylinder or other pressurized storage vessel) that retains a 0 mol% parahydrogen synthetic gas, for example. The source of the first reference fluid 3 can be at least one storage vessel (*e.g.,* a cylinder or other pressurized storage vessel) that retains a reference gas, such as a 25 mol% p-H2 hydrogen gas, for example.

The source of a sample fluid 7 can include at least one storage vessel (*e.g.,* a cylinder or other pressurized storage vessel) that retains a hydrogen gas from a sample of liquid hydrogen produced from a liquid hydrogen production facility (*e.g.,* a hydrogen liquefaction plant, a hydrogen liquefaction system, *etc.*). In some embodiments a manifold can be connected between the source(s) of the sample gas(es) 7 and the analyzer 6 so that fluid from different storage vessels can be fed to the analyzer 6 for analysis more quickly and easily. In some configurations, the sources of the first and second calibration fluids 5a and 5b can also be fluidly connected to such a manifold as well.

A conduit arrangement 4 can be provided to fluidly connect the analyzer 6 to the different sources of fluid. A manifold (not shown) can be provided as part of the conduit arrangement so that multiple different sample storage vessels can be fluidly connected to the analyzer for providing a quicker process for adjusting which sample fluid is fed to the analyzer for analysis.

For example, the conduit arrangement 4 can include a reference fluid feed conduit 4a that is positioned to fluidly connect the source of a reference fluid 3 with a reference fluid evaluation chamber 6b that is positioned within a housing 6a of the analyzer 6. The reference fluid feed conduit 4a can include a valve V that can be adjusted between closed and open positions in some embodiments. The conduit arrangement can also include a fluid analyzing feed conduit 4b that is fluidly connected to a comparative fluid evaluation chamber 6c that is positioned within a housing 6a of the analyzer.

The fluid analyzing feed conduit 4b can include a flow meter or other sensor for determining a flow rate of the fluid fed to the comparative fluid evaluation chamber 6c *via* the fluid analyzing feed conduit 4b. The reference fluid feed conduit 4a can also include a flow meter or other sensor for determining a flow rate of the fluid fed to the reference fluid evaluation chamber 6b *via* the reference fluid feed conduit 4b. These flow meter sensors can be communicatively connected to a computer device CD of the analyzer 6 for use in evaluation of data obtained by at least one sensing element 6d that is operatively connected to the comparative fluid evaluation chamber 6c and the reference fluid evaluation chamber 6b. The one or more sensing elements 6d can be communicatively connected to the computer device CD of the analyzer 6 *via* a communicative connection CC for providing that data to the computer device CD.

For example, at least one sensing element 6d can be positioned between the comparative fluid evaluation chamber 6c and the reference fluid evaluation chamber 6b for sensing at least one parameter related to the fluid passed through the comparative fluid evaluation chamber 6c and how that fluid may differ from the reference fluid passed through the reference fluid evaluation chamber 6b. The temperatures of both of these chambers can also be maintained to be the same temperature of substantially the same temperature during this evaluation (*e.g.,* within 0.1°C or within 0.5°C of each other) to help facilitate an accurate sensing of the parameter(s). Temperature sensors can be positioned for detecting of the temperatures of the different chambers (which can also be referred to as measuring cells) for monitoring and controlling the temperatures of these chambers.

For example, a parameter that can be detected can be a change in resistance of a voltage or current that can be provided *via* resistors positioned in the comparative fluid evaluation chamber 6c and the reference fluid evaluation chamber 6b. The data concerning such a change in resistance can be utilized by the computer device CD to evaluate how the thermal conductivity of the fluid passed through the comparative fluid evaluation chamber 6c differs from the thermal conductivity of the fluid passed through the reference fluid evaluation chamber 6b for use in determining a parahydrogen content of the fluid passing through the comparative fluid evaluation chamber 6.

For instance, in some embodiments, the sensing element(s) 6d can include at least one Wheatstone bridge circuit. As the reference fluid and the comparative fluid being analyzed are passed through their respective chambers, the bridge can become unbalanced to cause a current to flow in a detector circuit. The amount of this current can be an indication of type of gas or the orthohydrogen and/or parahydrogen concentration(s) within the gas.

After the reference fluid is passed through the reference fluid chamber 6b, it can be vented or otherwise output *via* a reference fluid outlet conduit 6o that is fluidly connected to the reference fluid chamber 6b. After the comparative fluid is passed through the comparative fluid evaluation chamber, the fluid can also be output from the comparative fluid evaluation chamber 6c *via* a fluid outlet conduit 6o that is fluidly connected to the comparative fluid evaluation chamber 6c for being vented or otherwise processed downstream of the analyzer 6.

As noted above, in some embodiments, the analyzer 6 can be configured so that the at least one sensing element 6d provides data related to a difference in thermal conductivity between the reference fluid being passed through the reference fluid evaluation chamber 6b and the fluid that is to be analyzed being passed through the comparative fluid evaluation chamber 6c. The analyzer 6 can include a computer device CD that is connected to the at least one sensing element 6d to receive the data and determine a content of the sample fluid based on the data (*e.g.,* current of voltage that may be passed through a detection circuit of the sensing element(s), *etc*.)*,* calibration data, and a pre-defined thermal conductivity analysis method that is defined by code stored in memory of the analyzer 6 that a processor of the computer device CD of the analyzer can execute.

In some embodiments, the analyzer 6 can be or can include a thermal conductivity ortho/para hydrogen analyzer (*e.g.,* Teledyne 2000 Orth/Para hydrogen analyzer, a model 2000A-EU thermal conductivity analyzer provided by Teledyne Analytical Instruments, or similar type of analyzer, *etc*.)*.*

The conduit arrangement 4 can also include valves V to facilitate a control of fluid fed to the analyzer 6. For example, the conduit arrangement can include a sample feed conduit 4c that is fluidly connected to the fluid analyzing feed conduit 4b for feeding a fluid from a source of a sample fluid 7 to the comparative fluid evaluation chamber 6c. The sample feed conduit 4c can include a valve V that can be adjusted between an open position and a closed position to either feed a sample fluid from the source of the sample fluid 7 to the comparative fluid evaluation chamber 6c of the analyzer 6 or prevent feeding of that fluid to the analyzer 6.

The conduit arrangement can include a first calibration fluid feed conduit 4d that is fluidly connected to the fluid analyzing feed conduit 4b for feeding a fluid from a source of a first calibration fluid 5a to the comparative fluid evaluation chamber 6c. The first calibration fluid feed conduit 4d can include a valve V that can be adjusted between an open position and a closed position to either feed the first calibration fluid feed from the source of the first calibration fluid 5a to the comparative fluid evaluation chamber 6c of the analyzer 6 or prevent feeding of that fluid to the analyzer 6.

The conduit arrangement can include a second calibration fluid feed conduit 4e that is fluidly connected to the fluid analyzing feed conduit 4b for feeding a fluid from a source of the second calibration fluid 5b to the comparative fluid evaluation chamber 6c. The second calibration fluid feed conduit 4e can include a valve V that can be adjusted between an open position and a closed position to either feed the second calibration fluid feed from the source of the second calibration fluid 5b to the comparative fluid evaluation chamber 6c of the analyzer 6 or prevent feeding of that fluid to the analyzer 6.

The first reference fluid and the second calibration fluid can be the same type of fluid in some embodiments. For example, in some embodiments the first reference fluid can be a 25 mol% p-H2 hydrogen gas stored in a storage vessel (*e.g.,* cylinder) that has been stored at room temperature for a sufficient period of time to have reached equilibrium condition and be at a known concentration of 25 mol% p-H2 in the hydrogen gas. The second calibration fluid can also be a 25 mol% p-H2 hydrogen gas stored in a storage vessel (*e.g.,* cylinder). The second calibration fluid can be a hydrogen gas that has been stored at room temperature for a sufficient period of time to have reached equilibrium condition and be at a known concentration of 25 mol% p-H2 in the hydrogen gas in some embodiments.

The sample fluid(s) of the at least one source of sample fluid 7 can be hydrogen fluid obtained from one or more production runs of a hydrogen liquefaction system or other hydrogen production system. The sample fluid(s) can be obtained from produced liquid hydrogen that has subsequently warmed to a gas in some embodiments, for example. Each source of sample fluid 7 can be a vessel or other type of storage container or group of storage containers that can retain the sample fluid for being fed to the analyzer 6 *via* the conduit arrangement 4.

The second calibration fluid stored in the source of the second calibration fluid 5b can be stored within at least one storage vessel or other type of storage container that is fluidly connectable to the analyzer 6 *via* the conduit arrangement 4. The first reference fluid stored in the source of the first reference fluid 3 can be stored within at least one storage vessel or other type of storage container that is fluidly connectable to the analyzer 6 *via* the conduit arrangement 4 as well.

The first calibration fluid can be a synthetic gas that is formed to mimic the thermal conductivity of a hydrogen has that has a 0 mol% p-H2 content. This synthetic gas can be formed and subsequently stored in at least one storage vessel or other storage type of storage container that is fluidly connectable to the analyzer 6 *via* the conduit arrangement 4.

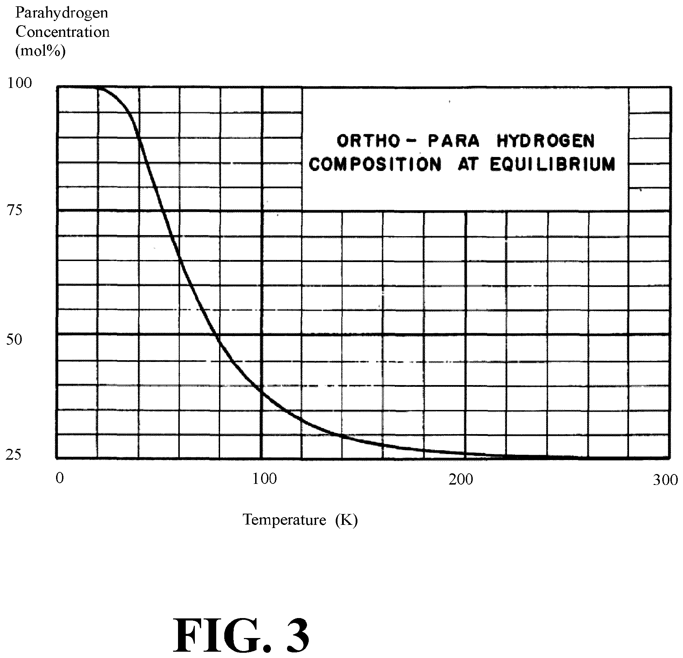

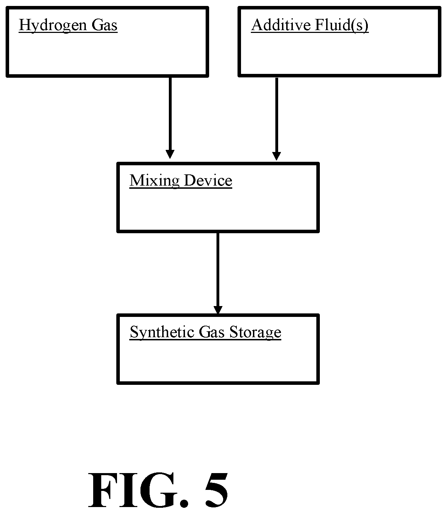

The forming of the synthetic gas that can be provided as the first calibration fluid can include mixing at least one thermally conductive additive fluid with a 25 mol% p-H2 hydrogen gas (e.g., a hydrogen gas that has been stored at room temperature sufficiently to reach an equilibrium at which it is 25 mol% p-H2 as can be appreciated from Figure 3, for example) to provide the synthetic gas that has the same or a substantially similar thermal conductivity as a 0 mol% p-H2 hydrogen gas (*e.g.,* a thermal conductivity that is within 5% of the thermal conductivity of a 0 mol% p-H2 hydrogen gas). Figure 5 illustrates an exemplary process for the creating of a synthetic gas.

As may best be seen from Figure 5, a hydrogen gas (*e.g.,* a 25 mol% p-H2 hydrogen gas) can be fed to a mixing device along with at least one additive fluid. The mixing device can be an inline-mixer, mixing vessel, or other type of mixing mechanism that can mix the fluids together, the mixed fluids can be output from the mixing device and fed into at least one storage vessel for storage of the formed synthetic gas (*e.g.,* at least one cylinder, at least one storage container, *etc*.)*.* The mixing can be performed so that the additive fluid is added to the hydrogen gas so that the thermal conductivity of the formed synthetic gas is equivalent to a thermal conductivity of a 0 mol% parahydrogen content hydrogen gas (*e.g.,* the thermal conductivity of the synthetic gas can be equal to or can be within a pre-defined tolerance of the thermal conductivity of a 0 mol% parahydrogen content hydrogen gas). For example, the formed synthetic gas can have a thermal conductivity that is within 0.1% or 0.01% of thermal conductivity of a 0 mol% parahydrogen content hydrogen gas. As another example, the formed synthetic gas can have a thermal conductivity that is within 1% or 0.5% of thermal conductivity of a 0 mol% parahydrogen content hydrogen gas.

For instance, in some embodiments the synthetic gas of the first calibration fluid that is to mimic the thermal conductivity of a 0 mol% p-H2 hydrogen gas can be formed by mixing helium gas as a thermally conductive additive fluid with a room temperature hydrogen gas that has been stored at room temperature sufficiently to be at an equilibrium condition such that the hydrogen gas is a 25 mol% p-H2 hydrogen gas to form the synthetic gas that mimics the thermal conductivity of a 0 mol% p-H2 hydrogen gas. In some embodiments, the helium can be mixed with the hydrogen gas so that the formed synthetic gas has between 0.5 mol% helium and 4 mol% helium. For example, embodiments can mix helium with the hydrogen gas to provide a synthetic gas that is between 1 mol% helium and 2 mol% helium with the balance being the 25 mol% p-H2 hydrogen gas. In some embodiments, the synthetic gas has been formed to provide a helium concentration of between 1.15 mol% and 1.30 mol% (*e.g.,* 1.17 mol% helium, 1.19 mol% helium, 1.20 mol% helium, 1.23 mol% helium, 1.25 mol% helium, 1.27 mol% helium, *etc.*)*.* In other embodiments, the synthetic gas has been formed to provide a helium concentration of between 1.17 mol% and 1.27 mol% (*e.g.,* 1.17 mol% helium, 1.18 mol% helium, 1.19 mol% helium, 1.20 mol% helium, 1.23 mol% helium, 1.24 mol% helium, 1.246 mol% helium, 1.25 mol% helium, 1.26 mol% helium, *etc.*)*.*

In yet other embodiments, a synthetic gas can be utilized that uses a different thermally conductive additive fluid or a different combination of thermally conductive additive fluids for mixing with a hydrogen gas to form the synthetic gas that is to mimic the thermal conductivity of a 0 mol% p-H2 hydrogen gas. For instance, the thermally conductive additive fluid can include, for example, nitrogen gas, krypton gas, argon gas, helium gas, or a combination of two or more of these gases. As yet another example, the thermally conductive additive fluid can include another suitable additive fluid for mixing with the 25 mol% p-H2 hydrogen gas to form a synthetic gas of the first calibration fluid that has the same thermal conductivity of a 0 mol% p-H2 hydrogen gas or a substantially similar thermal conductivity (*e.g.,* within 5% of the thermal conductivity of a 0 mol% p-H2 hydrogen gas, within 1% of the thermal conductivity of a 0 mol% p-H2 hydrogen gas, within 0.5% of thermal conductivity of a 0 mol% p-H2 hydrogen gas, within 0.1% of the thermal conductivity of a 0 mol% p-H2 hydrogen gas, *etc*.)*.*

The apparatus 1 for parahydrogen concentration analysis can be utilized to calibrate the analyzer 6 *via* use of the first and second calibration fluids and reference fluid and subsequently analyze one or more samples in serial or sequential fashion after the analyzer 6 is calibrated for a pre-selected time period or pre-selected number of analyzations before the analyzer 6 can again undergo calibration.

An example of a process in which the analyzer 6 can be calibrated and subsequently utilized for evaluation of the parahydrogen content and/or orthohydrogen content of a hydrogen fluid sample is shown in Figure 3. In first step S1, a synthetic gas can be created to have a thermal conductivity that mimics the thermal conductivity of a 0 mol% p-H2 hydrogen gas. As discussed above, this synthetic gas can be formed for use as the first calibration fluid stored in a source of a first calibration fluid 5a (*e.g.,* a storage vessel storing the synthetic gas). As noted above, the synthetic gas can be formed by mixing a 25 mol% p-H2 hydrogen gas with at least one other gas to form the synthetic gas that has the same or a substantially similar thermal conductivity to a 0 mol% parahydrogen hydrogen gas.

In a second step S2, the analyzer 6 can be calibrated. This calibration can involve providing the analyzer 6 with a first calibration point by passing the reference gas through the reference gas evaluation chamber 6b and passing the first calibration fluid (*e.g.,* a synthetic gas that is to mimic a 0 mol% p-H2 hydrogen gas) through the comparative fluid evaluation chamber 6c. This can be provided *via* closing of the valves V for the sample feed conduit 4c and the second calibration fluid conduit 4e and opening of the valve V of the first calibration fluid conduit 4d so that the first calibration fluid from the source of the first calibration fluid 5a can be provided to the analyzer 6. This feeding of the first calibration fluid and reference fluid to the analyzer 6 for being fed into and/or through the measurement cells of the analyzer 6 (*e.g.,* the reference fluid evaluation chamber 6b and comparative fluid evaluation chamber 6c) can occur for a pre-selected equilibrium time period to help ensure the fluid within the chambers are at an equilibrium condition to help provide an accurate analysis *via* the analyzer 6. The analyzer 6 can be provided with input to set its calibration to acknowledge that this first calibration fluid is a fluid that is at 0 mol% p-H2 hydrogen for the hydrogen gas as well *via* input devices (*e.g.,* buttons of a user interface of the analyzer, a computer device interface that is provided for a communicative connection with a computer device of the analyzer 6, *etc.*).

After the first calibration point is set on the analyzer *via* use of the first calibration fluid, the feeding of the first calibration fluid to the analyzer 6 can be stopped and the second calibration fluid can then be fed to the analyzer to provide a second calibration data point for the analyzer 6. For example, the valves V for the sample feed conduit 4c and the first calibration fluid conduit 4d can be closed and the valve V of the second calibration fluid conduit 4e can be opened so that the second calibration fluid from the source of the second calibration fluid 5b can be provided to the analyzer 6. This feeding of the second calibration fluid and reference fluid to the analyzer 6 for being fed into and/or through the measurement cells of the analyzer 6 (*e.g.,* the reference fluid evaluation chamber 6b and comparative fluid evaluation chamber 6c) can occur for a pre-selected equilibrium time period to help ensure the fluid within the chambers are at an equilibrium condition to help provide an accurate analysis *via* the analyzer 6. The analyzer 6 can then be provided with input to set its calibration to acknowledge that this second calibration fluid is a fluid that is at 25 mol% p-H2 hydrogen for the hydrogen gas as well *via* input devices (*e.g.,* buttons of a user interface of the analyzer, a computer device interface that is provided for a communicative connection with a computer device of the analyzer 6, *etc*.)*.*

This calibration using a synthetic gas as the 0 mol% p-H2 hydrogen gas and another calibration fluid (e.g., the 25 mol% p-H2 hydrogen gas) can help establish multiple calibration points for the analyzer's parahydrogen concentration detection analysis (*e.g.,* setting first and second calibration points for the analyzer's use in evaluation of sample fluids *via* its pre-defined analysis scheme) so that a more accurate assessment of the parahydrogen content within samples can be provided that may help ensure that parahydrogen content within samples are detected with improved accuracy for parahydrogen contents of 95 parahydrogen content and below to help detect samples that have hydrogen content that is at or above 95 mol% parahydrogen content more accurately and reliably. I have found that embodiments can be provided so that the analyzer 6 has a significantly higher degree of certainty that a sample being analyzed after calibration is above 95 mol% p-H2 hydrogen content *via* the calibration approach of this second step S2. Embodiments can be utilized to help better detect hydrogen fluid that is below 95 mol% parahydrogen content to indicate that such a product may not meet certain parahydrogen content specifications requiring a parahydrogen content of over 95 mol% and may also provide a more reliable certification for hydrogen product that is above 95 mol% parahydrogen content.

Further, I have found that this type of calibration approach can provide a much quicker, simpler, and less costly approach for calibration. For example, embodiments can be utilized without any need for use of an expensive catalyst tube apparatus. Also, the use of a synthetic fluid that can mimic a thermal conductivity of a 0 mol% p-H2 hydrogen gas has been found through experimentation to provide effective and reliable analysis for the p-H2 content of hydrogen gas samples taken from the liquid hydrogen production facility or from one or more liquid hydrogen storage and/or transport trailers. This lower cost - both in terms of capital cost and operational cost - as well as increased simplicity and improved operational flexibility (*e.g.*, no need to have to address issues related to the catalyst or its changing activity that can occur from use as the catalyst may become poisoned or occluded during use and require regeneration or replacement or from difficulties with not knowing the extent to which the hydrogen has reached its parahydrogen equilibrium or the temperature of the stored liquid hydrogen that can dictate the equilibrium p-H2 concentration value of the hydrogen.) help provide improved operational efficiency and improved operational reliability. Also, I have found that embodiments can permit more accurate and reliable certification of parahydrogen content in liquid hydrogen produced for aerospace applications and other applications in which high parahydrogen content specifications (*e.g.,* a parahydrogen content of at least 95 mol% p-H2 within the liquid hydrogen or hydrogen gas) can be requirements for at least some aerospace industry customers.

After the calibration of the analyzer 6 is completed, at least one sample from at least one source of a sample 7 can be fed to the analyzer for analyzing the parahydrogen content and/or orthohydrogen content of the sample in a third step S3. Each sample that is utilized in the analysis can be a sample from a liquid hydrogen production run that produced liquid hydrogen to help certify that parahydrogen content of the hydrogen.

For example, after calibration of the analyzer 6 is completed, the reference fluid and a sample fluid can be fed to the analyzer 6 for determination of a parahydrogen content and/or an orthohydrogen content of the sample (*e.g.,* a sample of liquid hydrogen, which has been recently vaporized to a gaseous state for the analysis). This can be provided *via* closing of the valves V for the first calibration fluid conduit 4d and the second calibration fluid conduit 4e and opening of the valve V of the sample fluid conduit 4c so that the sample fluid from the source of the sample fluid 7 can be provided to the analyzer 6. This feeding of the sample fluid and the reference fluid to the analyzer 6 for being fed into and/or through the measurement cells of the analyzer 6 (*e.g.,* the reference fluid evaluation chamber 6b and comparative fluid evaluation chamber 6c) can occur for a pre-selected equilibrium time period to help ensure the fluid within the chambers are at an equilibrium condition to help provide an accurate analysis *via* the analyzer 6. The analyzer 6 can subsequently measure the thermal conductivity difference between the sample fluid and the reference fluid to facilitate determination of a parahydrogen content and/or orthohydrogen content of the sample fluid *via* the at least one sensing element 6d and provide output that indicates the determined content value(s) for the parahydrogen content and/or orthohydrogen content of the sample fluid.

After a first sample is evaluated, the analyzer 6 may again be calibrated for another evaluation of a sample. Alternatively, the initial calibration may be acceptable for a number of evaluations utilizing additional samples. After a pre-selected time period or pre-defined number of sample analyses have been performed, the analyzer 6 may be calibrated again. In such a situation, the second step S2 of the process may again be repeated. In the event that there is an insufficient amount of synthetic gas available for use in calibration, the first step S1 may be performed before the second step S2 is performed for the calibration of the analyzer 6 for its subsequent use in evaluation of other samples.

In some embodiments, the computer device CD of the analyzer 6 can be a controller of the analyzer 6 or other computer device that is configured to receive data from the sensing element(s) 6d and other sensors (*e.g.,* flow meter sensors of conduits, temperature sensors of measuring cells, *etc*.) to analyze a parahydrogen content and/or orthohydrogen content of a fluid *via* a pre-defined analysis scheme defined in code of the memory of the computer device. The non-transitory memory of the computer device CD can be connected to a processor for execution of that code. The computer device can also include at least one interface for communicative connection to at least one user interface, which can be provided on a user interface element integrated into the housing 6a and/or a user device that can be communicatively connected to the analyzer's computer device *via* a network connection (*e.g.,* local area network connection, wireless local area network connection, *etc*.)*,* wired connection, or near field connection (*e.g.,* Bluetooth connection) *via* at least one transceiver that is communicatively connectable to the processor of the computer device CD. The user interface of the analyzer 6 that may be integrated into the housing 6a or otherwise connected to the computer device can include a display, a number of input elements (*e.g.,* buttons, knobs, keyboard, *etc*.) and can also be communicatively connectable to at least one user device (*e.g.,* laptop computer, tablet computer, workstation, data server, *etc.*) for communicating the analysis data that is generated by the computer device for storage, printing, or other user by an operator device or operator computer system.

I performed experimentation work to evaluate embodiments of the apparatus 1 and process. In the conducted experimentation work, I was able to determine a concentration of an additive gas (*e.g.,* helium) in a hydrogen gas that was a 25 mol% p-H2 hydrogen gas that provided analyzer readings of samples of liquid hydrogen of between 95 mol% p-H2 and 100 mol% p-H2 *via* calibration using a formed synthetic gas performed after I was satisfied that the concentration of the additive gas added into the hydrogen gas to form the synthetic gas was nominally correct to help confirm the proof of concept for an embodiment of the process and apparatus.

In other experimentation, a sample of liquid hydrogen having a known p-H2 content value can be provided for use in the experimentation work by aging liquid hydrogen stored in a tanker, or trailer, for at least two weeks to help ensure the hydrogen reaches its equilibrium p-H2 content value. The conducted experimentation has shown that sample analysis using an analyzer calibration *via* use of the first and second calibration fluids wherein one such fluid as a synthetic gas that mimicked a 0 mol% p-H2 hydrogen gas provided reliable and accurate determinations for parahydrogen contents of over 95 mol% parahydrogen contents of the samples.

I also conducted experiments of varying the mixture of hydrogen gas and at least one additive gas (*e.g.,* helium) to then achieve the target p-H2 measurement value to determine that the use of the synthetic gas as a proxy for a 0 mol% p-H2 gas can provide calibration of an analyzer 6 that results in the analyzer providing improved and reliable results in detection of parahydrogen contents of hydrogen that is above 25 mol% p-H2. I surprisingly found that this was possible because the thermal conductivity of hydrogen gas between 0 mol% parahydrogen and 100 mol% parahydrogen can be a linear function. Conducted experimentation I performed found that use of a two-point calibration at 0 mol% p-H2 and 25 mol% p-H2 was able to set a slope in pre-defined analysis modeling used by the analyzer that enabled the analyzer 6 to accurately extrapolate to the greater than 95 mol% p-H2 concentration value and the conducted experiments that were performed have proven the resulting calibration accurately measures the p-H2 content value in freshly vaporized liquid hydrogen trailer samples of known p-H2 values.

My conducted experimentation work has surprisingly found that forming a synthetic gas composition that has been experimentally shown to provide a thermal conductivity of a 0 mol% p-H2 (or 100 mol% orthohydrogen) hydrogen gas, which does not otherwise exist in nature. Embodiments of my apparatus 1, process, and calibration method can provide better results than a conventional catalyst tube based approach because it does not rely on unprovable assertions of the parahydrogen equilibrium value or the temperature of the liquid hydrogen from which the sample was taken to determine the p-H2 content value.

For example, in some experimental work that was performed, a tanker of liquid hydrogen that had been stored at normal ambient conditions for at least 2 weeks (a time sufficient for the stored liquid hydrogen to be at 100% equilibrium independent of any catalyst) was used to evaluate an embodiment of my process and apparatus. A temperature of the stored liquid hydrogen was obtained by measuring the vapor pressure of the liquid which is at vapor liquid equilibrium in the tanker. A published look-up table for parahydrogen content of liquid hydrogen at 100% equilibrium for different temperatures (*e.g.,* a lookup table based off of the contents shown in Figure 3) was then used to determine what the parahydrogen and orthohydrogen content of the liquid hydrogen was based on that measured temperature. Then, I utilized an embodiment of the apparatus and process to calibrate an analyzer 6 *via* use of the first and second calibration fluids (which included use of a synthetic 0 mol% parahydrogen gas and a 25 mol% parahydrogen gas as the first and second calibration fluids). After calibration, the analyzer was utilized to detect the parahydrogen content in a sample of the liquid hydrogen from the tanker to compare to the look-up table results with the results obtained *via* use of the calibration analyzer 6. This experimental work further showed that the analyzer 6 could accurately and reliably detect the parahydrogen content and/or orthohydrogen content of the liquid hydrogen sample after calibration *via* an embodiment of my process using an embodiment of my apparatus.

My work involved significant technical difficulty, however. For instance, the precise mix of additive gas to be included in a hydrogen gas to form the synthetic gas was extremely difficult to identify and create. For example, hydrogen gas can be difficult to purchase from commercial cylinder gas suppliers that has a sufficient purity content to help provide accurate content valuations (e.g., a concentration of hydrogen gas in which there is +/- 0.01 mol% helium), which is significantly higher purity than what can be obtained from a supplier, which normally provides hydrogen gas that is +/- 3 mol% of the requested content and about +/- 0.04 mol% helium.

In performing this experimental work I determined that utilization of a dilution system, or mixing system, that can mix gases in an analyzer panel to produce a target mix of gases at a desired composition similar to the processing shown in Figure 5 (and also discussed herein) can be an approach that can provide better and more reliable results in some situations to help provide a desired synthetic gas formation. The added cost associated with use of such a system is under $11,000 for providing 1-2 years of calibration support. This is far more cost-effective than a conventional catalyst based approach (which can cost over $40,000 in capital costs and also incur significant operational costs as noted herein) and can provide improved reliability of parahydrogen content evaluation work as discussed herein.

Embodiments of the apparatus for parahydrogen concentration analysis, process for parahydrogen concentration analysis, process for performing a calibration for parahydrogen concentration analysis, and/or process for forming a synthetic gas to mimic a 0 mol% parahydrogen content hydrogen gas, can be utilized in various different environments or industrial applications. For example, in some embodiments the process and/or apparatus may be utilized in a lab for conducting research. In other embodiments, the process and/or apparatus can be provided in conjunction with hydrogen production to provide certifications on parahydrogen content and/or orthohydrogen content of hydrogen produced by a facility or number of different facilities.

It should also be appreciated that other modifications can also be made to meet a particular set of criteria for different embodiments of the apparatus 1 or process. For instance, the arrangement of valves, flow meters, temperature sensors, other sensors, piping, and other conduit elements (*e.g.,* conduit connection mechanisms, tubing, seals, valves, *etc.*) for interconnecting different units of the apparatus for fluid communication of the flows of fluid between different elements can be arranged to meet a particular facility layout design that accounts for available area of the apparatus, sized equipment of the apparatus, and other design considerations. For instance, the size or type of the analyzer 6 and other equipment can be modified to meet a particular set of design criteria. As yet another example, each source of a fluid can include at least one storage vessel or container or a grouping of storage vessels or containers that can be fluidly connected to the analyzer 6. The sizing and type of the storage vessels or storage containers that can be utilized for sources of calibration fluids and/or samples can be any suitable sized and configured storage device that may be suitable for a particular set of design criteria for the apparatus 1.

It should also be appreciated that some embodiments of the process may adjust the calibration processing while still utilizing the synthetic gas as a calibration fluid. For example, in some embodiments the synthetic gas may be utilized as a second calibration fluid and a first calibration fluid can be used that is another hydrogen gas having another parahydrogen content (*e.g.,* the first calibration fluid can be a 25 mol% p-H2 hydrogen gas and the second calibration fluid can be the synthetic gas). In such an embodiment, the calibration can be performed so that the first calibration point entered into an analyzer for the first calibration gas can be a p-H2 content of that gas (*e.g.,* 25 mol% p-H2 hydrogen gas) and the second calibration point entered into the analyzer when the synthetic gas is utilized as the second calibration gas can be a 0 mol% p-H2 content.

As yet another example, it is contemplated that a particular feature described, either individually or as part of an embodiment, can be combined with other individually described features, or parts of other embodiments. The elements and acts of the various embodiments described herein can therefore be combined to provide further embodiments. Thus, while certain exemplary embodiments of the process, apparatus, system, and methods of making and using the same have been shown and described above, it is to be distinctly understood that the invention is not limited thereto but may be otherwise variously embodied and practiced within the scope of the following claims.

## Claims

1. A process for parahydrogen concentration analysis, the process comprising:
creating a synthetic gas to mimic a 0 mole percent (mol%) parahydrogen content hydrogen fluid such that the synthetic gas has a thermal conductivity that is equivalent to a thermal conductivity of a hydrogen fluid having a 0 mol% parahydrogen content;
calibrating an analyzer using the synthetic gas as a first calibration fluid and a second calibration fluid during the calibrating of the analyzer; and
after the calibrating of the analyzer, feeding a reference gas to the analyzer and a fluid of a sample of hydrogen fluid to the analyzer for determination of a parahydrogen content of the sample and/or an orthohydrogen content of the sample.

2. A process according to Claim 1, wherein the thermal conductivity of the synthetic gas is either equal to the thermal conductivity of the hydrogen fluid having the 0 mol% parahydrogen content, or is within 0.5%, e.g., within 0.1%, of the thermal conductivity of the hydrogen fluid having a 0 mol% parahydrogen content.

3. A process according to Claim 1 or Claim 2, wherein the analyzer is a thermal conductivity analyzer.

4. A process according to any of the preceding claims, wherein the creating of the synthetic gas comprises:
mixing a 25 mol% parahydrogen content hydrogen gas with helium gas to form the synthetic gas such that the synthetic gas has a helium content of between 0.5 mol% helium and 4 mol% helium and a balance of the synthetic gas being the 25 mol% parahydrogen content hydrogen gas.

5. A process according to Claim 4, wherein the helium content of the synthetic gas is between 1.15 mol% helium and 1.30 mol% helium, e.g., between 1.17 mol% helium and 1.27 mol% helium.

6. A process according to any of Claims 1 to 3, wherein the creating of the synthetic gas comprises:
mixing a 25 mol% parahydrogen content hydrogen gas with at least one additive fluid to form the synthetic gas, the at least one additive fluid including one or more of: nitrogen gas, krypton gas, argon gas, helium gas, or a combination thereof.

7. A process according to any of Claims 1 to 3, wherein the creating of the synthetic gas comprises:
mixing a hydrogen gas with at least one additive fluid to form the synthetic gas.

8. A process for performing a calibration for parahydrogen concentration analysis, the process comprising:
creating a synthetic gas to mimic a 0 mole percent (mol%) parahydrogen content hydrogen fluid such that the synthetic gas has a thermal conductivity that is equivalent to a thermal conductivity of a hydrogen fluid having a 0 mol% parahydrogen content; and
calibrating an analyzer using the synthetic gas as a first calibration fluid and a second calibration fluid.

9. A process according to Claim 8, wherein the calibrating of the analyzer using the synthetic gas as the first calibration fluid and the second calibration fluid includes:
feeding of the first calibration fluid and a reference fluid to the analyzer for being fed into and/or through measurement cells of the analyzer for a pre-selected equilibrium time period for establishing a first calibration data point for the analyzer; and
after the first calibration point is set on the analyzer *via* use of the first calibration fluid, stopping the feeding of the first calibration fluid to the analyzer and subsequently feeding the second calibration fluid and the reference fluid to the analyzer for a pre-selected equilibrium time period for establishing a second calibration data point for the analyzer.

10. A process according to Claim 9, wherein the calibrating of the analyzer using the synthetic gas as the first calibration fluid and the second calibration fluid also includes:
entering input into the analyzer to define a parahydrogen content of 0 mol% parahydrogen for the first calibration fluid while the first calibration fluid is fed to the analyzer to establish the first calibration data point; and
entering input into the analyzer to define a parahydrogen content of the second calibration fluid while the second calibration fluid is fed to the analyzer to establish the second calibration data point.

11. A process according to Claim 10, wherein the second calibration fluid is a hydrogen fluid and the parahydrogen content of the second calibration fluid is 25 mol% parahydrogen.

12. A process according to any of Claims 8 to 11, wherein the creating of the synthetic gas comprises:
mixing a 25 mol% parahydrogen content hydrogen gas with at least one additive fluid to form the synthetic gas, wherein the at least one additive fluid optionally including one or more of: nitrogen gas, krypton gas, argon gas, helium gas, or a combination thereof.

13. A process according to Claim 12, wherein the helium content of the synthetic gas is between 1.15 mol% helium and 1.30 mol% helium, e.g., between 1.17 mol% helium and 1.27 mol% helium.

14. An apparatus for parahydrogen concentration analysis, the apparatus comprising:
an analyzer;
a source of a reference gas that is fluidly connectable to the analyzer;
a source of a first calibration gas that is fluidly connectable to the analyzer, the first calibration gas being a synthetic gas that has a content of hydrogen and at least one additive fluid to mimic a 0 mole percent (mol%) parahydrogen content hydrogen fluid such that the synthetic gas has a thermal conductivity that is equivalent to a thermal conductivity of a hydrogen fluid having a 0 mol% parahydrogen content; and
a source of a second calibration fluid comprising hydrogen that is fluidly connectable to the analyzer.

15. An apparatus according to Claim 14, wherein the thermal conductivity of the synthetic gas is within 1% of the thermal conductivity of the hydrogen fluid having the 0 mol% parahydrogen content and the analyzer is a thermal conductivity analyzer.
